# EUROPEAN PATENT APPLICATION

(11) **EP 4 252 615 A1**
(43) Date of publication of application: **04.10.2023**
(21) Application number: 23157979.8
(22) Date of filing: 22.02.2023
(51) Int. Cl.: A61B 1/00, A61B 17/00

(54) **ALBARRAN AND SHAFT FOR AN ALBARRAN**

(30) Priority: 30.03.2022 US 202263325374 P
(71) Applicant: Olympus Winter & Ibe GmbH, 22045 Hamburg (DE)
(72) Inventor: Schmuck, Irina, 25746 Heide (DE); Schulz, Kevin Alexander, 22889 Wilstedt (DE); Schröder, Bastian, 22589 Hamburg (DE); Rühs, Andreas, 22926 Ahrensburg (DE)
(74) Representative: Hoener, Matthias

(57) **Abstract**

The invention relates to a shaft and an albarran which can be manufactured in a particularly simple manner and are particularly easy and time-efficient to clean. This is achieved in that a shaft (23) or an optics guide tube and at least one pull wire tube guide are formed in one piece. Due to this one-piece design of the shaft (23), the long closed pull wire tubes can be eliminated by design. Due to the open design of the shaft (23) according to the invention, the shaft (23) can be cleaned in a particularly simple and time-efficient manner. In addition, the manufacture of the shaft (23) is particularly simple.

## Description

The invention relates to a shaft for an albarran according to claim 1. Furthermore, the invention relates to an albarran according to claim 10.

Albarrans are used to support operations or treatments with surgical instruments such as endoscopes, resectoscopes, cystoscopes or similar. An albarran can be used, for example, to angle a flexible forceps inside a patient in a targeted and controlled manner. For this purpose, the albarran, just like an endoscope for example, has a rod-like or tube-like shaft, which is to be guided into the patient's body with a distal end. At a proximal end of the albarran, outside the patient, the shaft is connected to a main body. Via this main body, further instruments or tools, such as optics, wires or the like, can be guided through the shaft into the patient via various openings or ports.

At the distal end of the shaft, the albarran has a lever, the so-called albarran lever. This lever is designed to be movable and can be actuated or pivoted via a toggle on the main body. For this purpose, the lever is mechanically coupled to the toggle along the shaft. This mechanical coupling can be either a rod or a pull wire. Usually, the lever is connected to the toggle via two pull wires. It is conceivable that both pull wires serve equally for pivoting the lever back and forth or that the two pull wires can be used to effect various movements of the lever via the toggle.

The pull wires are passed from the proximal end to the distal end of the shaft, each through a pull wire tube. These pull wire tubes are aligned parallel to the shaft and welded to the outer wall of the shaft. The shaft, which can also serve to guide the optics, among other things, is also referred to as the optics guide tube in this context. The diameter of this optics guide tube or shaft is considerably larger than that of the two pull wire tubes.

By guiding the pull wires inside the pull wire tubes, the wires are guided on the one hand, and on the other hand, kinking of the wires during their transverse movement to actuate the lever is avoided.

In order to seal the main body or the drive body, which is connected to the pull wire tubes, against any fluids, it is known to fill the thin pull wire tubes with grease. This creates a sealing grease barrier between the fluid distally and the main body proximally. This filling creates long as well as thin channels or annular spaces filled with grease inside the pull wire tubes.

This known design of the stem has several disadvantages. Due to the increased requirements, the durability of the grease barrier in the pull wire tubes cannot always be guaranteed. Another disadvantage of the known shaft can be seen in the long weld seams between the pull-wire tubes and the optics guide tube, since their manufacture is associated with increased costs. It has also been shown that the long gaps between the pull wire tubes and the optics guide tube increase cleaning time and effort. An improvement in this respect would result in an optimized product for the customer.

On this basis, the invention is based on the problem of creating a shaft and an albarran that can be manufactured in a particularly simple manner and are particularly easy and time-efficient to clean.

A solution to this problem is described by the features of claim 1. Accordingly, it is provided that the shaft or optics guide tube and at least one pull wire tube guide are formed in one piece. By this one-piece design of the shaft, the long closed pull wire tubes can be eliminated by design. Due to the open design of the shaft according to the invention, the shaft can be cleaned in a particularly simple and time-efficient manner. In addition, the manufacture of the shaft is particularly simple.

Preferably, the invention provides that the shaft is formed as a profiled tube, wherein the at least one pull wire tube guide is a bulge parallel to a longitudinal axis of the profiled tube and the bulge thereby represents a cross-sectional extension of the, preferably concentric, profiled tube. In particular, it is provided that the profiled tube for receiving two pull wires has two pull wire tube guides formed as bulges, which are oriented parallel to each other with respect to the longitudinal axis of the profiled tube. The profile tube is designed in such a way that it serves equally to accommodate an instrument, in particular the optics, and the two pull wires. The pull wires are fixed in their bulges by the optics during use of the shaft. This can prevent the wires from kinking during treatment. Removing the optic from the socket after treatment is complete exposes the pull wires, thereby obtaining the open and thus easy-to-clean design of the shaft. The fact that this shaft no longer includes long welds in the longitudinal direction makes it particularly easy to manufacture. In addition, no long gaps are formed, which are particularly time-consuming to clean.

A further preferred embodiment of the invention may provide that a size or a radius of curvature or a shape of the at least one bulge corresponds to a size or a radius of curvature or a shape of the at least one pull wire tube, in particular is slightly larger. The bulges are dimensioned in such a way that the pull wires can be deposited in or pushed into these bulges and can be moved transversely back and forth parallel to the longitudinal axis of the shaft during treatment without major frictional or sliding resistance.

According to the invention, it is conceivable that the at least one bulge partially encloses the pull wire, preferably over an angle of 45° to 270°, in particular over an angle of 90° to 180°. By this partial enclosure of the pull wires by the bulges, the pull wires are already held or fixed in position before the insertion of the optics. This can help to simplify the assembly of the entire shaft.

A further aspect according to the invention can be seen in that a respective tube is arranged at a distal end and/or at a proximal end of the profiled tube. This at least one tube may be concentric and have the same radius of curvature as the profiled tube. For a mechanical connection, it is conceivable that the tubes are welded with one of their end faces to the end faces of the distal and/or proximal ends of the profiled tube. This welding can be done butt to butt and therefore turns out to be simple. At the proximal end of the shaft, the tube serves to form a firm connection with the, in particular conventional, main body. At the distal end of the shaft, the tube positioned there can serve to accommodate the albarran lever and its attachment. Furthermore, the tube or tubes serve to guide the pull wires. Namely, the pull wires are guided past the outer wall of the tube(s), whereby additional kink protection can also be achieved.

Preferably, it is also conceivable that the proximal and/or the distal end of the profiled tube or of the at least one tube has an at least substantially concentric tab whose diameter is smaller than the diameter of the profiled tube and of the tube. This tab can serve to connect or weld the profiled tube to the at least one tube. Through this tab, the profiled tube and the at least one tube can be plugged together and then welded. In this way, welding can be performed in a predefined and particularly simple manner.

An albarran for solving said problem has the features of claim 10. Such an albarran has an albarran lever and a main body with a drive body. The albarran lever is movable via at least one pull wire through a toggle on the drive body. Thereby, the at least one pull wire is supported in at least one pull wire tube guide and optics within a tube-like shaft according to at least one of claims 1 to 9. Due to the pull wire tube guide according to the invention, which is formed as a bulge in a profiled tube of the shaft, on the one hand the albarran can be cleaned particularly easily and quickly and produced in a simple and thus efficient manner. The interaction of an instrument or the optic, which is pushed into the profiled tube, and the pull wires allows them to be protected against kinking. After the optic has been removed from the shaft and the pull wires have been exposed, the profile tube or shaft can be cleaned particularly time-efficiently and easily due to its open design. The albarran described herein with the shaft according to the invention does not have any welds aligned parallel to a longitudinal axis of the shaft, which prevents the formation of long gaps or other areas that are difficult to access. The open design of the shaft also makes it possible to achieve the necessary cleanliness with little effort. For this reason, the albarran claimed here is particularly well suited for a wide range of reprocessing operations.

A preferred embodiment of the present invention is explained in more detail below with reference to the drawing. In this show:
- Fig. 1: a schematic representation of a prior art albarran,
- Fig. 2: a view of a distal end of the albarran shown in Fig. 1,
- Fig. 3: a cross-sectional view of a shaft, and
- Fig. 4: a perspective view of a distal end of the shaft.

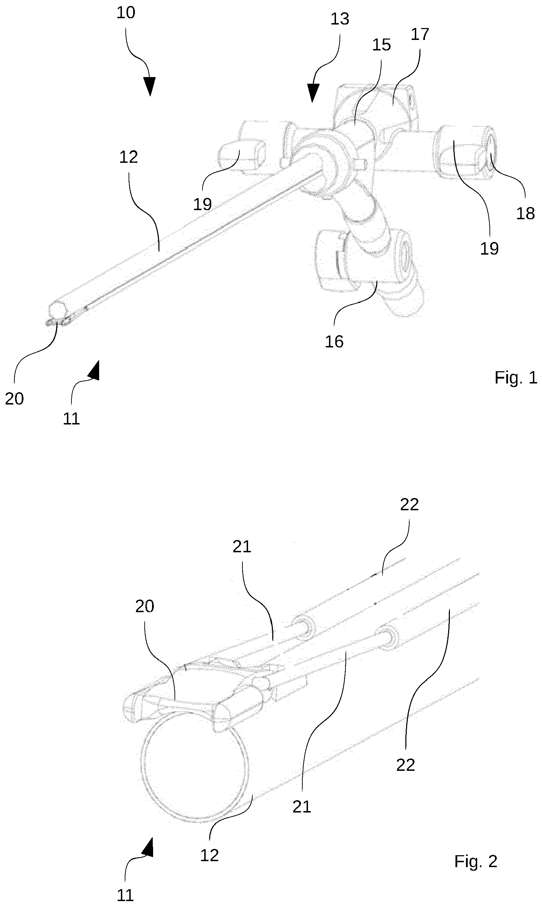

Fig. 1 shows a known albarran 10 according to the prior art. Reference is made here only to the features which are relevant to the description of the present invention. For a description of all features as well as the functionality of the albarran 10 according to Fig. 1, reference is made to the relevant prior art.

A main body 15 is arranged at a proximal end 13 of a shaft 12 of the albarran 10. This main body 15 is directly connected or connectable to the shaft 12. While for a treatment or operation of a patient the elongated shaft 12 is guided with a distal end 11 into an interior of the patient's body, the main body 15 remains outside the body and serves for providing and operating various tools or aids for performing the operation. For this purpose, the main body 15 has associated therewith, for example, a tube-like port 16 through which tools or other aids not shown can be guided through the shaft 12 to the distal end 11 of the albarran 10 to the site of the operation during the operation.

Furthermore, the main body 15 has a drive body 17. A toggle axis 18 of a toggle 19 leads through this drive body 17 perpendicular to a longitudinal axis of the albarran 10 or the shaft 12. By actuating this lever-like toggle 19 or by rotating the toggle 19 about the toggle axis 18, an albarran lever 20 can be actuated at the distal end 11 of the albarran 10. This albarran lever 20 serves as an auxiliary tool during the operation or treatment. By actuating this lever 20, other tools not shown which have been introduced into the body by other surgical instruments, such as endoscopes or cystoscopes, can be operated in an assisting manner. For example, by angling the albarran lever 20 clockwise, flexible forceps can be moved inside the body.

For the actuation of the albarran lever 20, the drive body 17 or the toggle 19 is motion-coupled with one or two pull wires 21. It is known to guide these pull wires 21 outside the shaft 12. In this case, pull wires 21 are guided through a respective tube 22, which are positioned on opposite sides of a wall of the shaft 12. For illustrative purposes, Fig. 2 shows the shaft 12 with the prior art albarran lever 20. From this figure, it is apparent that the tubes 22, also referred to as pull wire guides, extend parallel to the shaft 12 from the proximal end 13 to the distal end 11 or albarran lever 20. There, the pull wire 21 is coupled to the albarran lever 20 such that actuation of the lever 20 is possible. At a proximal end of the pull wires 21, these are connected in the drive body 17 or with a pull wire driver.

By operating the toggle 19 about the toggle axis 18, the at least one pull wire 21 is tensioned so that the albarran lever 20 rotates about a pivot axis. As soon as the toggle 19 is turned in an opposite direction, the mechanical tension on the pull wire 21 is released and the albarran lever 20 moves back to its original position. The toggles 19 are assigned to both sides of the power unit 17.

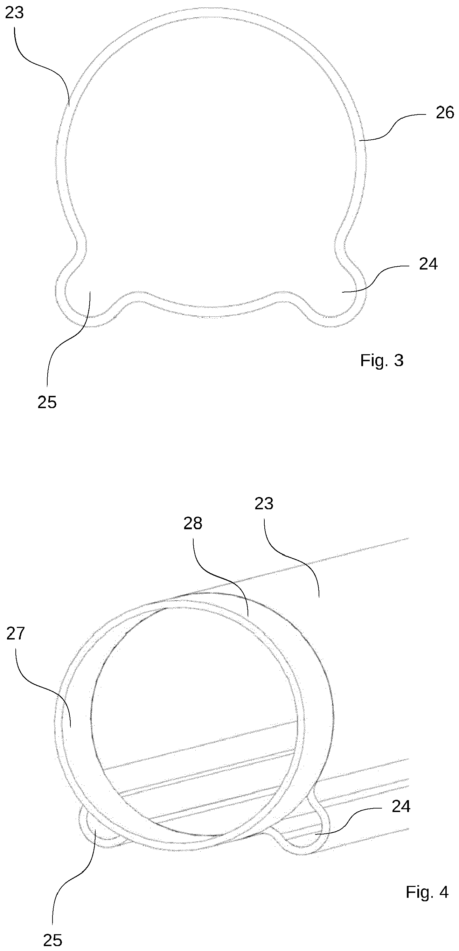

In contrast to the prior art, the invention provides for the pull wires 21 to be guided within a shaft 23 of the albarran 10. In Fig. 3, a cross-section of the shaft 23 according to the invention is shown. This shaft 23 is formed as a profiled tube with two bulges 24, 25. These bulges 24, 25 in a wall 26 of the shaft 23 are aligned parallel to a longitudinal axis of the shaft 23 and serve as a guide for the pull wires 21. The bulges 24, 25 at least partially form a receptacle with a circular cross-section. Thereby, this circular cross-section can enclose an angle of 45° - 270°, in particular 90° - 180°. As a result, the two pull wires 21 can be guided through or placed in the two bulges 24, 25 without much effort. Fixation of the pull wires 21 within the bulges 24, 25 can be achieved by means of a further instrument, such as optics, which is guided through the shaft 23. In this case, the further instrument corresponds with its outer circumference to the inner diameter of the shaft 23. As a result, the pull wires 21 are held in their bulges 24, 25. This prevents the pull wires 21 from buckling.

This open and one-piece design of the shaft 23 together with the bulges 24, 25 and the pull wire guides allows the albarran 10 to be easily assembled and cleaned in a very simple and time-efficient manner. The previously necessary greasing of the pull-wire guides is also not necessary with this profiled tube. After completion of the treatment, the instrument or optic is pulled out of the shaft 23, exposing the two pull wires 21, which can also be cleaned in a simple manner. Similarly, it is conceivable that the shaft 23 is simply uncoupled from the main body 15 and pulled away from the instrument or optic and the pull wires 21.

In order to stabilize the pull wires 21 at the proximal as well as distal ends of the shaft 23, it is conceivable to attach a small tube 27 to these ends of the shaft 23. This tube 27 has the same radius of curvature as the shaft 23, and the thickness of a wall 26 of the tube 27 is also preferably the same as the wall thickness of the shaft 23, so that both on the outer side and on the inner side the tube 27 fits seamlessly to the shaft 23. However, the tube 27 is concentric and therefore does not have the bulges 24, 25.

Preferably, it is provided that the one tube 27 or the two tubes 27 are welded tightly to the ends of the shaft 23. This circumferential weld seam is particularly easy to produce without great effort. When the two pull wires 21 now emerge from the bulges 24, 25 at the distal and proximal ends, respectively, they are guided along an outer wall 28 of the tubes 27. By this guiding along, the pull wires 21 are already fixed in the bulges 24, 25. The additional instrument or optic within the shaft 23 now also serves to prevent kinking of the pull wires 21.

The tube 27 can vary in length. In particular, it is conceivable that the tube 27 is such that the lever can also be attached to its outer wall 28. Also by means of the tube 27, the shaft 23 according to the invention retains its open as well as one-piece shape, which has proven to be particularly advantageous during manufacture, during use as well as during cleaning.

In addition to the embodiment example shown here, it is also conceivable that the shaft 23 has only one bulge 24, 25 in which only one pull wire 21 is guided. Furthermore, it is also conceivable that the shaft 23 has more than two bulges 24, 25.

At the proximal end of the shaft 23, the pull wires 21 emerge from the bulges 24, 25 and can be coupled to the main body or the drive body as before. Also, as before, at the distal end of the shaft 23, the pull wires 21 are usable with a lever to move the shaft in relation to the application.

### List of reference signs:

- 10: Albarran
- 11: Distal end
- 12: Shaft
- 13: Proximal end
- 15: Main body
- 16: Port
- 17: Drive body
- 18: Toggle axis
- 19: Toggle
- 20: Albarran lever
- 21: Pull wire
- 22: Tube
- 23: Shaft
- 24: Bulge
- 25: Bulge
- 26: Wall
- 27: Tube
- 28: Exterior wall

## Claims

1. Shaft (23) for an albarran (10) at the distal end of which an Albarran lever (20) and at the proximal end of which a main body (15) with a drive body (17) can be arranged, wherein the albarran lever (20) is movable via at least one pull wire (21) by means of a toggle (19) on the drive body (17) and the at least one pull wire (21) is mounted in at least one pull wire guide and an optical system is mounted within the tubular shaft (23), **characterized in that** the shaft (23) and the at least one pull wire guide are formed in one piece.

2. Shaft (23) for an albarran (10) according to claim 1, **characterized in that** the shaft (23) is formed as a profiled tube, wherein the at least one pull wire guide is a bulge (24, 25) parallel to a longitudinal axis of the profiled tube and the bulge (24, 25) thereby represents a cross-sectional extension of the, preferably concentric, profiled tube.

3. Shaft (23) for an albarran (10) according to claim 2, **characterized in that** the profiled tube for receiving two pull wires (21) has two pull wire guides formed as bulges (24, 25) which are oriented parallel to each other and to the longitudinal axis of the profiled tube.

4. Shaft (23) for an albarran (10) according to claim 2, **characterized in that** a size or a radius of curvature or a shape of the at least one bulge (24, 25) corresponds to a size or a radius of curvature or a shape of the at least one pull wire (21), in particular is slightly larger.

5. Shaft (23) for an albarran (10) according to claim 2, **characterized in that** the at least one bulge (24, 25) partially encloses the pull wire (21), preferably over an angle of 45° to 270°, in particular over an angle of 90° to 180°.

6. Shaft (23) for an albarran (10) according to one of the preceding claims, **characterized in that** a tube (27) is arranged at a distal end and/or at a proximal end of the profiled tube, respectively.

7. Shaft (23) for an albarran (10) according to claim 6, **characterized in that** the tube (27) is concentric and has the same radius of curvature as the profiled tube.

8. Shaft (23) for an albarran (10) according to claim 6 or 7, **characterized in that** the tube (27) is weldable to the profiled tube.

9. Shaft (23) for an albarran (10) according to any one of claims 6 to 8, **characterized in that** the proximal and/or the distal end of the profiled tube or of the at least one tube (27) has an at least substantially concentric tab whose diameter is smaller than the diameter of the profiled tube and of the tube (27).

10. Albarran (10) comprising an albarran lever (20) and a main body (15) comprising a power body (17), wherein the albarran lever (20) is movable via at least one pull wire (21) through a toggle (19) on the drive body (17) and the at least one pull wire (21) is supported in at least one pull wire guide and an optic within a tubular shaft (23) according to at least one of claims 1 to 9.
